(19) Europäisches Patentamt
European Patent Office
Office européen des brevets

(11) Numéro de publication : 0 361 578 B1

# (12) FASCICULE DE BREVET EUROPEEN

(45) Date de publication du fascicule du brevet : **30.12.92 Bulletin 92/53**

(51) Int. Cl.$^5$ : **C07C 17/00**

(21) Numéro de dépôt : **89202303.7**

(22) Date de dépôt : **14.09.89**

(54) **Procédé pour la fabrication d'hydrocarbure fluoré.**

(30) Priorité : **26.09.88 FR 8812643**

(43) Date de publication de la demande : **04.04.90 Bulletin 90/14**

(45) Mention de la délivrance du brevet : **30.12.92 Bulletin 92/53**

(84) Etats contractants désignés : **AT BE CH DE ES FR GB GR IT LI LU NL SE**

(56) Documents cités : **BE-A- 858 768**

(73) Titulaire : **SOLVAY**
**Rue du Prince Albert, 33**
**B-1050 Bruxelles (BE)**

(72) Inventeur : **Franklin, James**
**Rue Edouard Olivier, 28**
**B-1170 Bruxelles (BE)**
Inventeur : **Janssens, Francine**
**Koningslosteenweg, 106**
**B-1800 Vilvoorde (BE)**

(74) Mandataire : **Nichels, William et al**
**Solvay Département de la Propriété Industrielle Rue de Ransbeek, 310**
**B-1120 Bruxelles (BE)**

EP 0 361 578 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé pour la fabrication d'un hydrocarbure fluoré par réaction du fluorure d'hydrogène avec un hydrocarbure chloré insaturé en présence d'un composé de l'étain et d'un composé organophosphoré.

Le procédé de fabrication du 1-chloro-1,1-difluoréthane par réaction du fluorure d'hydrogène avec le chlorure de vinylidène en présence d'un composé de l'étain, tel que le tétrachlorure d'étain, est connu notamment par le brevet belge 858 768 au nom de Solvay & Cie.

Par ailleurs, la demande de brevet européen EP 0 187 643 divulgue d'une façon générale des procédés de fabrication d'hydrocarbures fluorés au départ d'hydrocarbures chlorés par réaction avec du fluorure d'hydrogène en présence d'un composé de l'étain et d'un additif choisi parmi des composés contenant de l'oxygène ou de l'azote; parmi ceux-ci sont notamment cités l'eau, l'eau oxygénée, des composés organiques oxygénés, tels que des alcools, cétones, acides carboxyliques, aldéhydes, éthers, esters et composés époxy; l'ammoniac ($NH_3$) et des composés organiques azotés, tels que pyridine et amines.

Les additifs connus de l'art antérieur présentent toutefois une stabilité peu élevée dans le milieu réactionnel et/ou réduisent l'activité du composé de l'étain, lorsqu'ils sont mis en oeuvre en présence de fluorure d'hydrogène et d'un composé chloré tel que le chlorure de vinylidène, ce qui compromet l'efficacité de ces procédés.

L'invention quant à elle concerne un procédé pour la fabrication d'un hydrocarbure fluoré à l'aide d'un additif qui ne présente plus ces inconvénients. On a en effet trouvé un procédé qui présente une bonne efficacité et dans lequel l'activité du catalyseur mis en oeuvre est accrue et/ou la stabilité des additifs est élevée et dans lequel peu d'oligomères sont formés et la sélectivité en produits intéressants est élevée, ce qui est accompagné d'une réduction de la pollution par les effluents. En outre, lorsqu'on opère dans un réacteur en continu, le taux des purges du milieu réactionnel liquide est réduit, ce qui limite donc les pertes en fluorure d'hydrogène et en catalyseur. Par ailleurs, du fait de l'absence d'eau ou de composés générant ce produit, on observe une nette diminution de la corrosion du réacteur puisqu'il n'y a pas formation d'acide chlorhydrique aqueux.

L'invention concerne à cet effet un procédé pour la fabrication d'un hydrocarbure fluoré par réaction du fluorure d'hydrogène avec un hydrocarbure chloré insaturé en phase liquide en présence d'un catalyseur constitué par un composé de l'étain et d'un additif constitué par un composé organophosphoré.

Le composé organophosphoré mis en oeuvre dans le procédé de l'invention est généralement choisi parmi les phosphites, phosphines ou phosphates organiques. Habituellement, il répond aux formules générales (A) ou (B) :

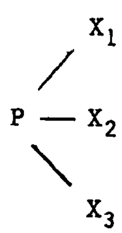 (A) ou 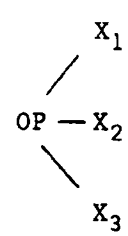 (B)

dans laquelle P représente l'élément phosphore

O l'élément oxygène

et $X_1$, $X_2$, $X_3$ représentent, indépendamment les uns des autres, les radicaux OR ou R, dans lesquels R représente l'hydrogène, un halogène, un groupement aliphatique saturé, substitué ou non, linéaire ou branché, comptant de 1 à 12 atomes de carbone, ou un groupement aromatique substitué ou non, comptant de 5 à 10 atomes de carbone.

Avantageusement, R représente un halogène tel que le chlore, un groupement aliphatique saturé non substitué linéaire ou branché, comptant de 1 à 8 atomes de carbone ou un groupement aromatique substitué ou non comptant au moins 6 et de préférence 6 à 8 atomes de carbone.

De manière préférée, R représente le chlore, un groupement aliphatique saturé non substitué linéaire ou branché comptant de 1 à 5 atomes de carbone ou un groupement phényl.

De manière particulièrement préférée, le composé organophosphoré mis en oeuvre est choisi parmi le phosphite de triéthyle, le phosphite de triméthyle, le phosphite de tri-n-butyle, le phosphite de triisopropyle, le phosphite de diéthyle, la tri-n-butylphosphine, la triphénylphosphine, la chlorodiphénylphosphine, la triphénylphosphine oxyde.

De bons résultats ont été obtenus avec le phosphite de triéthyle, le phosphite de triméthyle, le phosphite de tri-n-butyle, le phosphite de triisopropyle, la chlorodiphénylphosphine et la triphénylphosphine oxyde. De

très bons résultats ont été obtenus avec le phosphite de triéthyle.

Le procédé selon l'invention fait usage d'un catalyseur constitué par un composé de l'étain. Comme composé de l'étain on utilise de préférence les halogénures tels que les chlorures et les fluorures, les oxydes et les oxyhalogénures et de préférence les chlorures et fluorures d'étain. Le tétrachlorure d'étain s'est révélé particulièrement intéressant.

La quantité d'additif organophosphoré mis en oeuvre dans le procédé selon l'invention est généralement comprise entre 0,01 et 5 moles par mole de catalyseur. De préférence, on opère entre 0,05 et 3 moles d'additif par mole de catalyseur et de manière particulièrement préférée entre 0,1 et 2 moles d'additif par mole de catalyseur.

La quantité de catalyseur présente dans le procédé peut varier entre de larges limites. Elle correspond généralement à un poids compris entre 0,1 et 30 % de celui du milieu réactionnel et de préférence entre 0,5 et 15 % du poids de celui-ci.

La quantité de fluorure d'hydrogène mis en oeuvre dans le procédé peut également varier entre de larges limites. Elle est généralement comprise entre 1 et 5 moles par mole d'hydrocarbure chloré et de préférence entre 1,5 et 4 moles d'hydrocarbure chloré mis en oeuvre.

La température à laquelle la réaction est réalisée, est généralement comprise entre 30 et 180°C et de préférence entre 40 et 120°C. La pression à laquelle la réaction est réalisée est choisie de manière à maintenir le milieu réactionnel sous forme liquide. Elle est le plus souvent comprise entre 2 et 50 bars et varie selon la température du milieu réactionnel.

Le procédé selon l'invention s'applique à la fabrication de tout hydrocarbure fluoré ou chlorofluoré à partir de l'hydrocarbure chloré insaturé correspondant et en particulier à la fabrication du 1,1-dichloro-1-fluoréthane et du 1-chloro-1,1-difluoréthane à partir du chlorure de vinylidène.

Le procédé de l'invention peut être réalisé dans tout réacteur ou appareillage permettant de réunir les conditions décrites ci-avant. Le procédé peut être réalisé en batch ou en continu.

L'additif organophosphoré peut être mis en oeuvre de diverses manières, ainsi il peut être introduit tel quel dans le réacteur avant la réaction, ou être dissous dans l'hydrocarbure chloré et être introduit avec celui-ci concomitamment, ou être dissous dans un produit intermédiaire de la réaction.

Dans le cas de la réaction à partir du chlorure de vinylidène en présence de phosphite de triéthyle, de bons résultats ont été obtenus suite à l'introduction tel quel de l'additif dans le réacteur avant la réaction et suite à l'introduction de l'additif solubilisé dans le chlorure de vinylidène.

Les exemples qui suivent illustrent l'invention.

## Exemple 1

On utilise un autoclave de 0,5 l, équipé d'une soupape de sécurité, d'un système d'introduction des réactifs, d'une prise d'échantillon liquide, d'un manomètre, d'un agitateur et d'un système de prélèvement constitué d'un barboteur suivi d'un réservoir d'eau.

On introduit dans cet autoclave 0,05 mole de phosphite de triéthyle.

On fait le vide (environ 20 mm Hg) sur l'autoclave et on le refroidit à -40°C par trempage dans un mélange éthanol/$CO_2$. Puis on introduit dans l'autoclave 1 mole de chlorure de vinylidène par aspiration.

On introduit ensuite 0,05 mole de tétrachlorure d'étain et immédiatement après 3 moles de fluorure d'hydrogène.

On chauffe l'autoclave à 60°C et la température du milieu réactionnel est maintenue constante à 60°C pendant toute la durée de la réaction.

L'évolution de la pression est suivie au cours du temps, jusqu'à ce qu'elle atteigne 17,5 bars absolus.

On refroidit alors brutalement l'autoclave à -20°C.

La réaction a duré 5,5 heures.

Le taux de transformation du chlorure de vinylidène (VC2) est de 98,5%

$$(\text{taux de transformation} = \frac{\text{VC2 mis en oeuvre en mole} - \text{VC2 retrouvé en mole}}{\text{VC2 mis en oeuvre en mole}}).$$

L'analyse de l'ensemble des produits obtenus est donnée au tableau 1 ci-après.

$$(\text{Sélectivité} = \frac{\text{produit dosé en mole}}{\text{somme des produits dosés en mole}}).$$

Tableau 1

| Produits obtenus : sélectivité | |
|---|---|
| 1,1,1-trifluoréthane | 0,1 |
| 1-chloro-1,1-difluoréthane | 33,5 |
| 1,1-dichloro-1-fluoréthane | 61,1 |
| 1,1,1-trichloréthane | 4,9 |
| % molaire | |
| oligomères (exprimés en perte de VC2 mis en oeuvre) % | 0,37 |

Exemple 2R

On opère dans des conditions identiques à celles de l'exemple 1, mais sans ajouter du phosphite de triéthyle ni aucun autre additif au milieu réactionnel.

La réaction a duré 5 heures.

Le taux de transformation du chlorure de vinylidène est de 99,3%.

L'analyse de l'ensemble des produits obtenus est donnée au tableau 2 ci-après.

Tableau 2

| Produits obtenus : sélectivité | |
|---|---|
| 1,1,1-trifluoréthane | 1,5 |
| 1-chloro-1,1-difluoréthane | 39,6 |
| 1,1-dichloro-1-fluoréthane | 54,7 |
| 1,1,1-trichloréthane | 3,2 |
| % molaire | |
| oligomères (exprimés en perte de VC2 mis en oeuvre) % | 0,87 |

La comparaison des exemples 1 et 2R montre que l'addition de phosphite de triéthyle a pour effet de réduire la formation des oligomères de façon avantageuse.

Exemples 3 et 4

On répète l'exemple 1 mais en mettant en oeuvre comme additif 0,01 mole de phosphite de triéthyle pour

l'exemple 3 et 0,025 mole de phosphite de triéthyle pour l'exemple 4.

L'analyse de l'ensemble des résultats obtenus est donnée au tableau 3 ci-après.

Tableau 3

| | Exemple 3 | Exemple 4 |
|---|---|---|
| Produits obtenus : sélectivité | | |
| 1,1,1-trifluoréthane | 1,2 | 0,1 |
| 1-chloro-1,1-difluoréthane | 42,7 | 41,3 |
| 1,1-dichloro-1-fluoréthane | 51,8 | 53,8 |
| 1,1,1-trichloréthane | 3,7 | 4,4 |
| % molaire | | |
| oligomères (exprimés en perte de VC2 mis en oeuvre) % | 0,47 | 0,30 |
| durée de la réaction heure | 3,5 | 2,5 |
| taux de transformation du chlorure de vinylidène % molaire | 99,8 | 99,0 |
| activité du catalyseur | accroissement | fort accroissement |

Les exemples 3 et 4 montrent que de faibles quantités de phosphite de triéthyle suffisent pour réduire la fabrication d'oligomères.

Exemples 5 et 6

On répète l'exemple 1, mais en mettant en oeuvre comme additif 0,05 mole de phosphite de triméthyle pour l'exemple 5, 0,05 mole de phosphite de tri-n-butyle pour l'exemple 6.

L'analyse de l'ensemble des résultats obtenus est donnée au tableau 4 ci-après.

Tableau 4

| | Exemple 5 | Exemple 6 |
|---|---|---|
| Produits obtenus : sélectivité<br><br>1,1,1-trifluoréthane<br>1-chloro-1,1-difluoréthane<br>1,1-dichloro-1-fluoréthane<br>1,1,1-trichloréthane<br>% molaire | <br><br>< 0,1<br>29,1<br>65,2<br>5,1 | <br><br>< 0,1<br>38,3<br>56,9<br>4,2 |
| oligomères (exprimés en perte de VC2 mis en oeuvre) % | 0,45 | 0,55 |
| durée de la réaction heure | 3 | 5,75 |
| taux de transformation du chlorure de vinylidène % mole | 99,1 | 99,5 |
| activité du catalyseur | accroissement | inchangée |

Exemples 7 et 8

On répète l'exemple 1, mais en mettant en oeuvre comme additif 0,05 mole de triphénylphosphine oxyde pour l'exemple 7 et 0,05 mole de chlorodiphénylphosphine pour l'exemple 8.

L'analyse de l'ensemble des résultats obtenus est donnée au tableau 5 ci-après.

Tableau 5

| | Exemple 7 | Exemple 8 |
|---|---|---|
| Produits obtenus : sélectivité | | |
| 1,1,1-trifluoréthane | 0,6 | 0,5 |
| 1-chloro-1,1-difluoréthane | 39,7 | 40,8 |
| 1,1-dichloro-1-fluoréthane | 54,8 | 54,1 |
| 1,1,1-trichloréthane | 4,5 | 4,2 |
| % molaire | | |
| oligomères (exprimés en perte de VC2 mis en oeuvre) % | 0,36 | 0,35 |
| durée de la réaction heure | 5,25 | 5,50 |
| taux de transformation du chlorure de vinylidène % mole | 99,6 | 99,7 |
| activité du catalyseur | inchangée | inchangée |

Les exemples 5, 6, 7 et 8 montrent que différents composés organophosphorés peuvent être mis en oeuvre de façon avantageuse.

Exemple 9R

On répète l'exemple 1 mais en mettant en oeuvre comme additif 0,05 mole de méthyléthylcétone.
La réaction a duré 3,75 heures.
Le taux de transformation du chlorure de vinylidène est de 99,2 %.
L'analyse de l'ensemble des produits obtenus est donnée au tableau 6 ci-après.

Tableau 6

| | |
|---|---|
| Produits obtenus : sélectivité | |
| 1,1,1-trifluoréthane | 0,7 |
| 1-chloro-1,1-difluoréthane | 39,7 |
| 1,1-dichloro-1-fluoréthane | 54,3 |
| 1,1,1-trichloréthane | 4,5 |
| % molaire | |
| oligomères (exprimés en perte de VC2 mis en oeuvre) % | 0,68 |

La comparaison des exemples 2R et 9R montrent que l'addition de méthyléthylcétone n'apporte qu'une très faible réduction du taux de formation des oligomères.

Par ailleurs, la méthyléthylcétone est instable dans le milieu et entraîne la formation de sous-produits indésirables.

## Revendications

1. Procédé pour la fabrication d'un hydrocarbure fluoré par réaction du fluorure d'hydrogène avec un hydrocarbure chloré insaturé en phase liquide en présence d'un catalyseur constitué par un composé de l'étain et d'un additif caractérisé en ce que l'additif est un composé organophosphoré.

2. Procédé selon la revendication 1, caractérisé en ce que le composé organophosphoré est un phosphite, une phosphine ou un phosphate organique.

3. Procédé selon la revendication 2, caractérisé en ce que le composé organophosphoré répond aux formules générales (A) ou (B) :

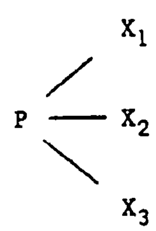 (A) ou 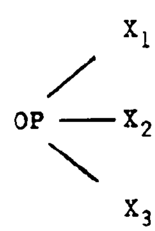 (B)

dans laquelle P représente l'élément phosphore

O représente l'élément oxygène

et $X_1$, $X_2$, $X_3$ représentent, indépendamment les uns des autres, les radicaux OR ou R dans lesquels R représente l'hydrogène, un halogène, un groupement aliphatique saturé, substitué ou non, linéaire ou branché, comptant de 1 à 12 atomes de carbone ou un groupement aromatique substitué ou non, comptant de 5 à 10 atomes de carbone.

4. Procédé selon la revendication 3, caractérisé en ce que le composé organophosphoré mis en oeuvre est le phosphite de triéthyle.

5. Procédé selon l'une quelconque des revendications précédentes, caractérisé en ce qu'on met en oeuvre de 0,01 à 5 moles d'additif par mole de catalyseur.

6. Procédé selon l'une quelconque des revendications précédentes, caractérisé en ce que le catalyseur mis en oeuvre est le tétrachlorure d'étain.

7. Procédé selon l'une quelconque des revendications précédentes caractérisé en ce que l'hydrocarbure chloré mis en oeuvre est le chlorure de vinylidène.

## Patentansprüche

1. Verfahren zur Herstellung eines fluorierten Kohlenwasserstoffs durch Umsetzung von Fluorwasserstoff mit einem ungesättigten chlorierten Kohlenwasserstoff in flüssiger Phase in Anwesenheit eines Katalysators, der durch eine Zinnverbindung und einem Zusatzstoff gebildet wird, dadurch gekennzeichnet, daß der Zusatzstoff eine organo-phosphorhaltige Verbindung ist.

2. Verfahren nach Anspruch 1, dadurch gekennzeichnet, daß die organo-phosphorhaltige Verbindung ein organisches Phosphit, Phosphin oder Phosphat ist.

3. Verfahren nach Anspruch 2, dadurch gekennzeichnet, daß die organo-phosphorhaltige Verbindung den allgemeinen Formeln (A) oder (B) entspricht:

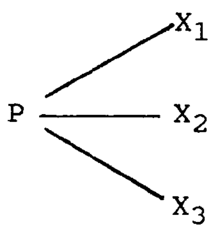 (A) oder 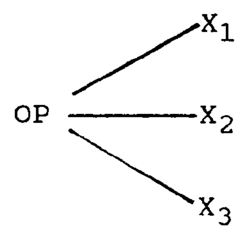 (B)

worin P das Element Phosphor darstellt
O das Element Sauerstoff darstellt
und $X_1$, $X_2$, $X_3$ unabhängig voneinander die Radikale OR oder R darstellen, in denen R Wasserstoff, ein Halogen, eine gesättigte, substituierte oder nicht-substituierte, lineare oder verzweigte aliphatische Gruppierung mit 1 bis 12 Kohlenstoffatomen oder eine substituierte oder nicht-substituierte aromatische Gruppierung mit 5 bis 10 Kohlenstoffatomen darstellt.

4. Verfahren nach Anspruch 3, dadurch gekennzeichnet, daß die eingesetzte organo-phosphorhaltige Verbindung Triethylphosphit ist.

5. Verfahren nach einem der vorhergehenden Ansprüche, dadurch gekennzeichnet, daß man 0,01 bis 5 Mol Zusatzstoff pro Mol Katalysator verwendet.

6. Verfahren nach einem der vorhergehenden Ansprüche, dadurch gekennzeichnet, daß der eingesetzte Katalysator Zinntetrachlorid ist.

7. Verfahren nach einem der vorhergehenden Ansprüche, dadurch gekennzeichnet, daß der eingesetzte chlorierte Kohlenwasserstoff Vinylidenchlorid ist.

## Claims

1. Process for the manufacture of a fluorinated hydrocarbon by the reaction of hydrogen fluoride with an unsaturated chlorinated hydrocarbon in the liquid phase in the presence of a catalyst consisting of a tin compound and in the presence of an additive, characterised in that the additive is an organophosphorus compound.

2. Process according to Claim 1, characterised in that the organophosphorus compound is an organic phosphite, phosphine or phosphate.

3. Process according to Claim 2, characterised in that the organophosphorus compound corresponds to the general formulas (A) or (B):

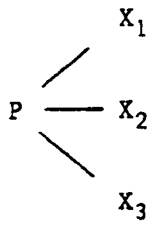 (A) or 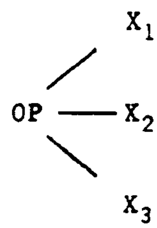 (B)

in which P represents the element phosphorus,

O represents the element oxygen and

$X_1$, $X_2$ and $X_3$ represent, independently of each other, the radicals OR or R, in which R represents hydrogen, a halogen, a substituted or unsubstituted saturated linear or branched aliphatic group having from 1 to 12 carbon atoms, or a substituted or unsubstituted aromatic group having from 5 to 10 carbon atoms.

4. Process according to Claim 3, characterised in that the organophosphorus compound used is triethyl phosphite.

5. Process according to any one of the preceding claims, characterised in that from 0.01 to 5 moles of additive are used per mole of catalyst.

6. Process according to any one of the preceding claims, characterised in that the catalyst used is tin tetrachloride.

7. Process according to any one of the preceding claims, characterised in that the chlorinated hydrocarbon used is vinylidene chloride.